# EUROPÄISCHE PATENTSCHRIFT

(11) **EP 0 442 137 B1**
(45) Veröffentlichungstag und Bekanntmachung des Hinweises auf die Patenterteilung: **14.02.1996**
(21) Anmeldenummer: 90125332.8
(22) Anmeldetag: 22.12.1990
(51) Int. Cl.: A61B 17/32, A61B 17/22

(54) **Vorrichtung zur Atherektomie**
Atherectomy device
Dispositif d'athérectomie

(30) Priorität: 14.02.1990 DE 4004507; 12.06.1990 DE 9006606 U; 07.04.1990 DE 9004074 U; 11.07.1990 US 551013
(43) Veröffentlichungstag der Anmeldung: 21.08.1991
(73) Patentinhaber: ANGIOMED GmbH & Co. Medizintechnik KG, D-76227 Karlsruhe (DE)
(72) Erfinder: Schnepp-Pesch, Wolfram, W-7505 Ettlingen (DE); Lindenberg, Josef, W-7500 Karlsruhe (DE); Starck, Erhard Dr., W-6242 Kronberg/Taunus (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 338 965
- EP-A- 0 352 872
- DE-A- 2 545 761
- DE-U- 8 702 530
- US-A- 2 730 101
- US-A- 4 273 128
- US-A- 4 679 557
- US-A- 4 770 174

## Beschreibung

Die Erfindung betrifft eine Vorrichtung nach dem Oberbegriff des Anspruchs 1.

Es ist eine derartige Vorrichtung zur Atherektomie bekannt, die an dem proximalen Ende einer als schraubenförmig gewickelter Draht ausgebildeten Antriebswelle zwei konisch aufeinander zu gerichtete Schneidmesser aufweist. Im Hinblick auf eine nicht eindeutige Terminologie hinsichtlich der Bezeichnung proximal und distal wird in der vorliegenden Anmeldung unter Berücksichtigung, daß rumpfwärts bzw. zum Herzen hin gelegene Teile einer Extremität durchgängig als "proximale" bezeichnet werden, das zum Herzen hin gelegene Ende der erfindungsgemäßen Vorrichtung bzw. eines Teils derselben als proximal und der zum Operateur hin gerichtete Teil als distaler bezeichnet.

Bei der bekannten Vorrichtung hat der distale Bereich der Schneiden aufgrund der zum proximalen Ende hin konischen Zurichtung einen erheblichen Durchmesser, wobei auch die Welle einen entsprechenden Durchmesser hat. Da die Welle durch einen Katheter geführt werden muß, muß dieser einen entsprechend noch größeren Durchmesser haben. Der durch die bekannte Vorrichtung zu schaffende Kanal durch eine Stenose oder einen Verschluß in einem Gefäß bestimmt daher die Größe der Punktionsstelle zum Einführen der Vorrichtung, die einen wesentlich größeren Querschnitt aufweisen muß als der im Gefäß freizulegende Kanal. Die durch Schraubenwicklung eines Drahtes gebildete Welle läßt sich aufgrund ihrer Stärke schlecht abdichten, so daß Blut aus dem Gefäß und distal aus der Vorrichtung austreten kann. Bei stärkerer Festklemmung eines Abdichtventils erhöht sich die Reibung derart, daß eine Beschädigung der Antriebswelle eintreten kann.

Weiterhin können Ablagerungen, wie thrombotische Okklusionen in Gefäßen oder dergleichen, durch ein in das Gefäß zum Verschluß beziehungsweise der Verengung hin geführtes drehbares Element abgetragen werden, wobei einerseits vorgeschlagen wird, eine Rotation im Bereich von 100.000 Umdrehungen pro Minute zu verwenden, um das Okklusionsmaterial zu pulverisieren. Es besteht die Gefahr, daß die pulverisierten Teilchen sich wieder an anderen Stellen, möglicherweise in wesentlich engeren, aber wichtigen Gefäßen, ablagern und dort zu Schädigungen führen können. Andere, mit geringer Drehzahl bis höchstens 500 Umdrehungen pro Minute umlaufende Vorrichtungen beschränken sich darauf, das gefäßverschließende Material zur Seite zu drängen. Hiermit kann keine zuverlässige dauerhafte Freilegung einer Stenose erreicht werden, von einer Öffnung einer Okklusion ganz zu schweigen. Es sind weiterhin Vorrichtungen bekannt, die verengendes Material im Gefäß in der einen oder anderen Form herausschneiden. Soweit hier ein drehender Katheter eingesetzt wird, so besteht die Gefahr, daß dieser bei seiner Drehung die Gefäßwand mitnimmt und so eine Verschlingung zu verursachen droht, so daß auch hier nur mit sehr geringen Geschwindigkeiten gearbeitet werden kann. Soweit das Abtragen durch an einer Welle durch einen Katheter geführte Arbeitswerkzeuge geschieht, so müssen diese mit Abstand zur Kathetermündung arbeiten, so daß gegebenenfalls durch den Katheter ausgeübter Sog zur Entfernung der abgetragenen Partikel nicht ausreicht, sämtliche Partikel mitzuziehen.

Die US-A-4 679 557 zeigt eine Vorrichtung zur Atherektomie mit einem Katheter mit einem hindurchgeführten länglichen Teil aus einem drehbaren Hohlelement und einem sich durch dieses hindurch erstreckenden Zugdraht, wobei am proximalen Ende des Hohlelements ein Arbeitselement angebracht ist. Das Hohlelement ist an seinem distalen Ende zum Drehantrieb des mit ihm verbundenen spreizbaren Elements mit einem motorischen Drehantrieb versehbar, und der Katheter ist in seinem distalen Endbereich mit einer Absaugeinrichtung verbindbar, mittels der Ablagerungen durch den Katheter hindurch absaugbar sind, die mittels des spreizbaren Elements von einer Gefäßwandung entfernt wurden (siehe Beschreibung, Spalte 1, Zeile 61-67). Die Verbindung von Hohl- und Arbeitselement ist eine Schwachstelle: es besteht die Gefahr, daS sie sich löst, wodurch das Arbeitselement vom Hohlelement getrennt wird. Dies ist für den Patienten höchst gefährlich.

Der Erfindung liegt daher die Aufgabe zugrunde, unter Vermeidung der vorgenannten Nachteile bei einer Atherektomie-Vorrichtung die Betätigung des Arbeitselements zu verbessern.

Erfindungsgemäß wird die genannte Aufgabe durch eine Atherektomie-Vorrichtung nach dem Oberbegriff des Anspruchs 1 gelöst, welche die kennzeichnenden Merkmale des Anspruchs 1 aufweist. In bevorzugter Weise kann vorgesehen sein, daß die Schneide(n) sich in der achsnahen Stellung im wesentlichen axial erstreckt (erstrecken).

Durch die Vorsehung von radial spreizbaren Elementen zum Abtragen der Beläge etc. wird es ermöglicht, diese zunächst in ihrer kontrahierten Stellung in das Gefäß einzuführen, so daß eine Punktion mit einem geringen Querschnitt notwendig ist. Andererseits können die Elemente am Operationsort radial gespreizt werden, so daß mit ihnen eine Atherektomie mit einem im Gefäß freigelegten Kanal durchgeführt werden kann, der einen wesentlich größeren Querschnitt als die notwendige Punktionsstelle aufweist. Hierdurch wird die Belastung des Patienten reduziert. Weiterhin wird das Arbeiten mit einer erfindungsgemäßen Vorrichtung erleichtert.

Grundsätzlich können die Elemente durch ein Spreizelement nach außen in ihre Schneid- bzw. Betriebsstellung gestellt werden. Nach einer solchen Ausgestaltung wird der Erweiterungsvorgang der Gefäßverengung auf jeden Fall von deren proximalem Ende her durchgeführt, wobei die Elemente zunächst in kontrahierter Vorrichtung durch einen in herkömmlicher Weise ausgebildeten Kanal dorthin bewegt wurden, bevor sie radial gespreizt werden. Grundsätzlich das gleiche Vorgehen kann auch vorgenommen werden, ist aber nicht notwendig, bei einer Ausgestaltung, bei der Endbereiche der Elemente axial aufeinander zu bewegbar sind. Bei der Ausgestaltung sind die Elemente elastisch flexibel ausgebildet und werden radial dadurch gespreizt, daß mit Axialabstand auf jeweils einem Element liegende Punkte axial aufeinander zu bewegt werden, wodurch die vorzugsweise drahtförmigen Elemente radial nach außen gebogen werden. Diese Ausgestaltung weist den Vorteil auf, daß keine sich nach außen erstreckenden freien Stirnseiten oder Enden der Elemente vorhanden sind. Während die Schneidelemente grundsätzlich aus Stahldraht bestehen können, sind sie insbesondere bei der vorbeschriebenen Ausgestaltung aus Nickeltitanlegierung, wie aus Nitinol, die gegenüber Stahldraht eine höhere Flexibilität aufweist. Es wird vorzugsweise Flachdraht verwendet, an dem die Schneiden geschliffen sind.

Die axiale Spannung der Elemente wird in der bevorzugten Ausgestaltung dadurch erreicht, daß die Elemente an Tragteilen ausgebildet sind, die an den mit Abstand zueinander angeordneten Verbindungsstellen zusammengehalten sind und daß die Verbindungsstellen axial aufeinander zu bewegbar sind. Die Elemente weisen vorzugsweise auf einem Teilbereich der Drahtteile durch Zuschleifen ausgebildete Schneiden oder Messer auf. Die Schneiden weisen dabei im wesentlichen in tangentiale Richtung eines durch die Drahtteile gebildeten Rotationskörpers. Das Spannen kann in konkreter Weise dadurch geschehen, daß der Zugdraht durch die Verbindungsstellen geführt ist und mittels ihm die Verbindungsstellen zueinander hinziehbar sind, wobei weiterhin vorgesehen sein kann, daß die Drahtteile im Bereich der Verbindungsstellen durch Hülsen zusammengehalten sind.

Eine weitere Ausbildung sieht vor, daß sich an die Elemente bzw. Drahtstücke proximal ein schraubenförmig gewickelter Hohldrahtansatz anschließt, wobei der distal ausgebildete Hohldraht eine Antriebswelle zum Drehantreiben der Schneiden bildet.

Der proximal zu den Elementen angeordnete Hohldrahtansatz trägt dazu bei, vor der Aufweitung des Gefäßlumens eine Rekanalisation zu schaffen, um den Aufweit- und Abtragvorgang durch die Elemente vom proximalen Ende der Verengung her vornehmen zu können.

Wenn gemäß weiterer bevorzugter Ausgestaltung vorgesehen ist, daß der Zugdraht die Elemente bzw. Einzeldrähte proximal überragt und an seinem überragenden Ende ein erweitertes Widerlager gegen eine distal ausgeübte Zugkraft befestigt ist und daß das Widerlager als Kugel ausgebildet ist, so kann durch Ziehen am Zugdraht in distaler Richtung über das Widerlager die Zugkraft auf den proximalen Zusammenführungs- bzw. Verbindungsbereich der gegebenenfalls Schneiden tragenden Einzeldrähte übertragen werden, so daß dieser Verbindungsbereich in dieser Weise zum distalen Verbindungsbereich der Einzeldrähte gezogen werden kann, wodurch diese sich - gegebenenfalls mit ihren Schneiden - radial nach außen stellen.

Während die Rekanalisation grundsätzlich in bekannter Weise durchgeführt werden kann, sieht eine bevorzugte Ausgestaltung vor, daß die erfindungsgemäße Vorrichtung hierzu eingesetzt werden kann, so daß vermieden wird, daß verschiedene Instrumente durch den Führungskatheter wiederholt ein- und ausgebracht werden müssen. Hierzu ist die am sich durch den proximalen Hohldrahtabschnitt hindurch erstreckenden Zugdraht ausgebildete Kugel oder allgemein ein Widerlager mit Schneidteilen versehen. Solche Schneidteile können durch auf der Kugel angebrachte Diamantsplitter, durch an dieser ausgefräste Schneiden oder auf dieser befestigte Schneidfolie gebildet sein.

In weiterer Ausgestaltung kann insbesondere vorgesehen sein, daß der Zugdraht als Hohldraht ausgebildet ist und sich dessen Hohlraum gegebenenfalls durch das Widerlager hindurch erstreckt. Bei einer solchen Ausgestaltung kann zunächst in völlig üblicher Weise ein herkömmlicher Führungsdraht gelegt werden, über den dann die erfindungsgemäße Vorrichtung eingeführt wird. Hierdurch wird neben einem antegraden Ausschälen des Gefäßes insbesondere ein retrogrades Freilegen unterstützt.

In einer anderen Ausgestaltung ist vorgesehen, daß die Vorrichtung bzw. die spreizbaren Elemente zangenartig ausgebildet ist. Dabei ergibt sich eine besonders einfache Realisierung, wenn die Schneiden angeschliffene Axialkanten der Zangenbacken sind.

Um abgetragene Elemente zur Kathetermündung zu bewegen, damit diese von dort anliegendem Unterdruck zuverlässig abgesaugt werden können, weist die Vorrichtung weiterhin ein drehbares Anschlußteil zum Drehantrieb der spreizbaren Elemente auf.

Durch die axial bewegliche Ausgestaltung des durch den Antrieb gedrehten Anschlußteils desselben für die die Ablagerung abtragenden Arbeitswerkzeuge (die in unterschiedlichster Weise ausgestaltet sein können) wird es möglich, die Arbeitswerkzeuge einerseits zum Abtragen des zu entfernenden Materials aus der Kathetermündung heraus in den Bereich der Stenose oder Okklusion einzuführen und andererseits rhythmisch die Arbeitswerkzeuge unter Aufrechterhaltung der Arbeitsdrehung zur Kathetermündung und gegebenenfalls teilweise in diese hinein zurückzubringen, so daß mit den Arbeitswerkzeugen mitgeführte Partikel zuverlässig durch den im Katheter oder im hohlen Führungsteil ausgeübten Unterdruck abgesaugt werden können.

Die durch den Antrieb getriebenen Arbeitswerkzeuge können in unterschiedlicher Weise ausgebildet sein. Es können spiralförmige Ausbildungen oder Körbchenausbildungen sein, bei denen also Abtragungselemente in Form von Drähten - stumpf oder zugeschliffen - in Axialrichtung auf dem Umfang eines fiktiven Rotationsellipsoiden angeordnet sind. Diese beiden Ausgestaltungen fangen in dem durch die Abtragungsdrähte beziehungsweise die Schrauben- oder Wendelausbildung gegebenen Bereich abgetragenes Material und können dieses daher bei ihrer Zurückbewegung zur Mündung des hohlen Führungsteils hin zuverlässig mitnehmen, so daß das abgetragene Material zuverlässig abgesaugt wird. Grundsätzlich sind aber auch andere Ausgestaltungen der Werkzeuge möglich.

Die axiale Hin- und Herbewegung des Anschlußteils des erfindungsgemäßen Antriebs und damit der mit diesem verbundenen Arbeitswerkzeuge erfolgt in einer bevorzugten Ausgestaltung manuell, wobei vorteilhafterweise vorgesehen ist, daß das drehbare Anschlußteil manuell axial hin- und herbewegbar ist. Hierdurch kann der Operateur die axiale Hin- und Herbewegung der Arbeitswerkzeuge in gewünschter Weise steuern und einstellen. Zur Erleichterung und Entlastung des Operateurs kann in alternativer Weise auch vorgesehen sein, daß das drehbare Anschlußteil motorisch axial hin- und herbewegbar ist. Der Axialantrieb kann dabei derart ausgebildet sein, daß eine axial fest, aber drehbar mit dem Anschlußteil und drehfest mit dem Gehäuse des Antriebs verbundene Hülse mit einer geschlossenen, in Form eines Meanders um den Umfang geführten Nut, in der die Nocken eines mit gegenüber der Winkelgeschwindigkeit des Anschlußteils geringer Winkelgeschwindigkeit umlaufenden Übertragungsteils eingreift. Die Hin- und Herbewegung kann von dem gleichen Motor abgeleitet sein, der die hochtourige Drehbewegung im Bereich von wenigen 100 bis 2000 Umdrehungen pro Minute des Anschlußteils und damit der Arbeitswerkzeuge bewirkt, indem zwischen der Abtriebswelle des Motors und dem Umsetzgetriebe zur Umsetzung eines Drehantriebs in die lineare Hin- und Herbewegung ein Übersetzungsgetriebe angeordnet ist, wie es beispielsweise grundsätzlich aus Uhrwerken bekannt ist.

Zum Anschluß der Arbeitswerkzeuge beziehungsweise derselben über eine durch das Führungsteil hindurchragenden Welle kann das Anschlußteil ein Kopplungselement beispielsweise in Form eines Luer-Loks oder dergleichen aufweisen. Das hohle längliche Führungsteil, insbesondere ein Katheter wird mit dem Antrieb fest dadurch verbunden, indem an der Aufnahmeeinrichtung für diesen eine in axialer Richtung beidseitig durch Schultern begrenzte nach innen gerichtete Umfangsnut ausgebildet ist, in die radial Flanschteile des Katheters oder mit diesem fest verbundene Elemente eingesetzt werden können.

Der Antrieb weist vorzugsweise einen Elektromotor auf und ist, um einerseits eine Unabhängigkeit vom Stromnetz zu gewährleisten, andererseits aus sicherheitstechnischen Gründen mit einem eigenen Energiespeicher versehen. Dieser kann ein aufladbarer Akkumulator oder aber eine Batterie sein. In beiden Fällen ist es vorteilhaft, weiterhin vorzusehen, daß die Anzeigeeinrichtung eine LCD-Anzeige ist. Hierdurch wird der Operateur rechtzeitig auf mangelnden Energievorrat des Energiespeichers und Abfall der Spannung hingewiesen, so daß er während einer Operation hiervon nicht überrascht wird, sondern rechtzeitig vorher den Energiespeicher austauschen kann. Vorzugsweise ist auch der Akkumulator auswechselbar in einem entsprechenden Fach im Gehäuse angeordnet. Eine weitere bevorzugte Ausgestaltung sieht vor, daß der Antrieb mit einem Geschwindigkeitsregler versehen ist. Weiterhin weist er selbstverständlich einen Ein- und Ausschalter auf.

Weitere Vorteile und Merkmale der Erfindung ergeben sich aus den Ansprüchen und aus der nachfolgenden Beschreibung, in der zwei Ausführungsbeispiele der Erfindung unter Bezugnahme auf die Zeichnung im einzelnen erläutert ist. Dabei zeigt:
- Figur 1: eine Seitenansicht, teilweise geschnitten, des Schneidbereichs der erfindungsgemäßen Vorrichtung in Einführungszustand;
- Figur 2: den Gegenstand der Figur 1 im Arbeitszustand;
- Figur 3: einen Schnitt entsprechend III-III der Figur 2;
- Figur 3a: einen Schnitt entsprechend der Figur 3 mit stumpfen Körbchendrähten;
- Figur 4: eine Gesamtdarstellung der erfindungsgemäßen Vorrichtung mit Antriebsteil;
- Figur 5: eine zangenartige Ausbildung einer erfindungsgemäßen Vorrichtung;
- Figur 6: ein weiteres Ausführungsbeispiel.

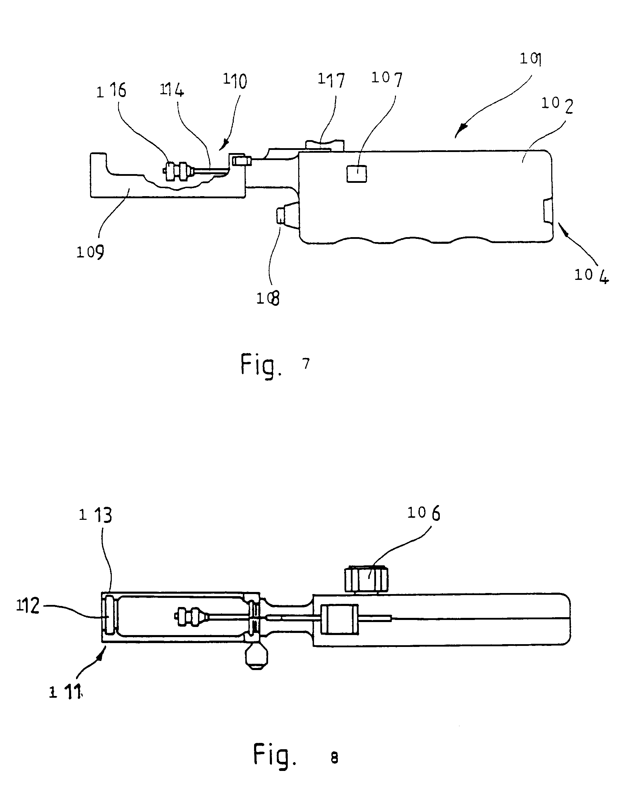

- Figur 7: eine Seitenansicht des erfindungsgemäßen Antriebs;

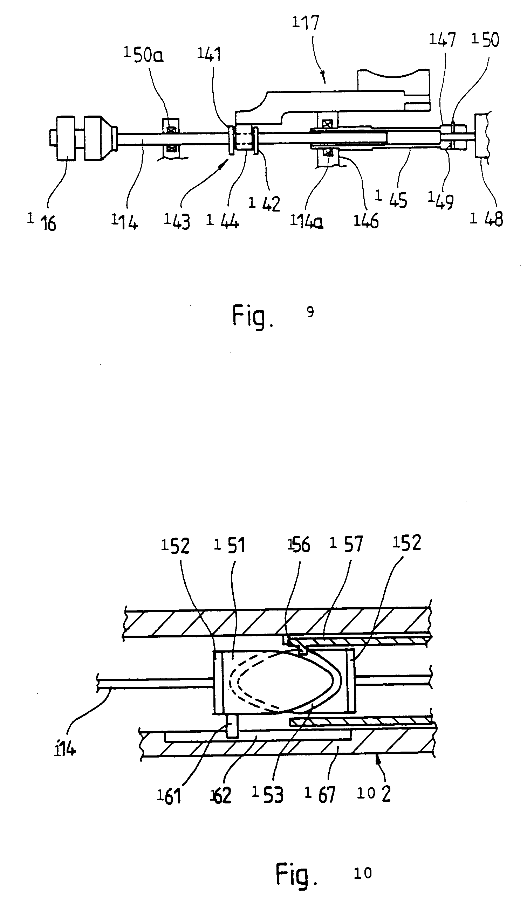

- Figur 8: eine Draufsicht auf den Antrieb entsprechend dem Teil II der Figur 1; und
- Figur 9: eine bevorzugte Ausführungsform der Kopplung des Linearantriebs mit dem mitteltourigen Drehantrieb.
- Figur 10: die schematische Darstellung eines Ausführungsbeispiels für einen motorischen hin- und hergehenden Linearantrieb des Anschlußteils

Die erfindungsgemäße Vorrichtung weist in der dargestellten Ausführungsform einen flexiblen Hohldraht 1 auf, der beispielsweise durch Wendeln von drei Litzen 2, 3, 4, gebildet ist. Der Hohldraht 4 ist durch einen Katheter 6 geführt. In einem als Zusammenführungsstelle 7 bezeichneten Bereich ist die Schraubenwicklung der Einzeldrähte 2, 3, 4 aufgehoben und diese sind im wesentlichen parallel zueinander axial geführt, bis sie in einem weiteren Zusammenführungsbereich 8, der mit Abstand zu dem erstgenannten angeordnet ist, wieder zusammengeführt und in Schraubgewindeführung zu einen weiteren Abschnitt 9 des Hohldrahtes 1 gebildet sind. An den Zusammenführungsstellen 7, 8 können Hülsen 11, 12 aufsitzen, die ein Aufdrehen der gewickelten Bereiche verhindern. Am proximalen Ende 13 kann ebenfalls eine Hülse 14 auf dem Hohldraht 1 aufgeklemmt sein.

Durch den gesamten Hohldraht 1 erstreckt sich frei und axial zu diesem verschiebbar ein Zugdraht 21. Daß aus dem proximalen Ende 13 des Hohldrahtansatzes 9 herausragende Ende des Zugdrahts 21 ist mit einem Widerlager in einer Kugel 22 versehen, deren Durchmesser zumindestens über dem Innendurchmesser des Hohldrahtansatzes 9 liegt und vorzugsweise dem Außendurchmesser des Hohldrahtansatzes 9 bzw. der Hülse 14 entspricht oder größer ist. Die Kugel 22 oder ein entsprechendes gegebenenfalls konisch ausgebildetes Widerlager kann mit Schneidkanten versehen sein, die entweder direkt an der Kugel ausgebildet sind, durch aufgebrachte Diamantsplitter oder dergleichen oder aber eine mit Mikroschneidkanten versehene Folie sind; insbesondere im letzteren Fall ist das Widerlager vorzugsweise als Kegel oder auch Zylinder ausgebildet.

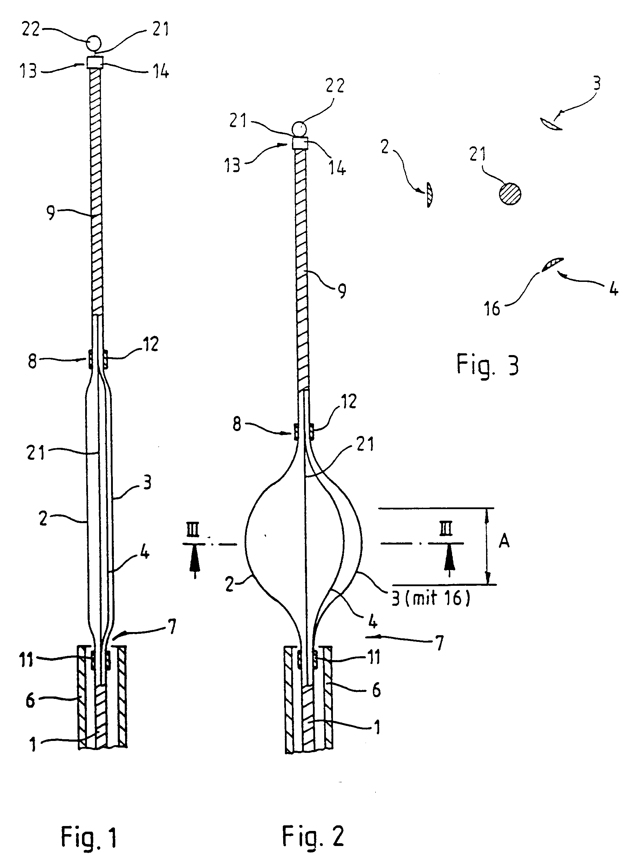

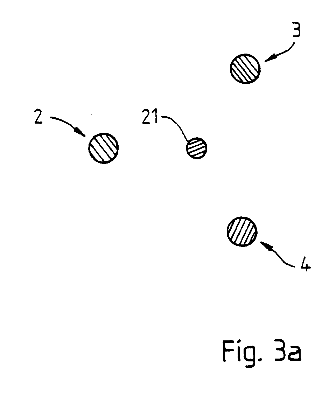

Die sich zwischen den Bereichen 7, 8 frei erstreckenden Einzeldrähte 2, 3, 4 sind zumindestens im Mittelbereich ihrer Längserstreckung A mit Schneiden 16 versehen. Die Schneiden 16 sind dabei im wesentlichen tangential zum zentralen Zugdraht 21 bzw. einen durch die Drähte 2, 3, 4 gebildeten Rotationsellipsoid gerichtet. Sie können dabei von einer Seite jedes Drahtes ausgebildet sein und zwar wenn nur eine Drehrichtung vorgesehen ist dann in Drehrichtung; vorzugsweise sind sie aber an zwei Seiten ausgebildet, so daß die erfindungsgemäße Vorrichtung mit zwei Drehrichtungen eingesetzt werden kann. Weiterhin liegen die Schneidkanten 16 radial gesehen (zum Draht 21 bzw. dem erwähnten Rotationskörper) möglichst außerhalb, wie dies in der Figur 3 dargestellt ist. Alternativ müßten die Drähte 2, 3, 4 des der durch sie gebildeten Körbchens keine Schneidkanten aufweisen, können vielmehr auch stumpf, beispielsweise aus Runddraht ausgebildet sein, wie dies in der Figur 3a dargestellt ist.

Wenn nun die Drähte 2 bis 4 mit ihren Schneidkantenbereichen 16 in der aus Figur 1 ersichtlichen unbelasteten Stellung, bei denen sie nur eine geringe Querschnittsfläche umschließen durch den Katheder 6 in den Bereich einer Stenose eines Gefäßes, gegebenenfalls nach Rekanalisation eingebracht wurde, so kann eine Atherektomie vorgenommen werden, indem der Zugdraht 21 vom distalen Ende der Vorrichtung her in distale Richtung gezogen wird. Hierzu werden die freigespannten Drähte 2 bis 4 axial belastet und ihre Enden axial gegeneinandergedrückt, wodurch die Mittelbereiche der Drähte nach außen gedrückt werden. Durch Rotation der Drähte 2 bis 4, gegebenenfalls durch einen am distalen Ende des Hohldrahtes 1 angreifenden motorischen - vorzugsweise batterie- oder akkubetriebenen - Antrieb können durch die Schneidkanten 16 Ablagerungen im Bereich der Stenose abgetragen werden und derart das Gefäß wieder weitgehend freigelegt werden, indem der Radius des durch die Drähte 2 bis 4 gebildeten Rotationsellipsoids stufenweise oder kontinuierlich bis zu dem gewünschten Umfang erweitert wird. Insbesondere kann eine Freilegung bis zu einem Querschnitt erreicht werden, der über dem Querschnitt des Einführungskatheters 6 liegt. Weiterhin wird durch die erfindungsgemäße Vorrichtung erreicht, daß trotz sehr kleiner Funktionstellen im Bereich von fünf Charrière oder "French" (5/3 ≈ ca. 1,7 mm) Gefäße bis zu einem Durchmesser von über zehn Charrière, teilweise bis zu 8 mm freigelegt werden können.

Die Rekanalisation einer gegebenenfalls vollständig geschlossenen Stenose kann in herkömmlich und an sich bekannter Weise erfolgen. Stattdessen kann, wenn das Teil 22, wie eine Kugel mit Schneidelementen versehen ist, die erfindungsgemäße Anordnung bis vor die Stenose vorgeschoben werden, wobei der Zugdraht 21 als Führungsdraht dienen kann. Auf jeden Fall wird, sobald die Kugel 22 unter der gleichen an der Stenose zur Anlage kommt der Zugdraht 21 in Rotation versetzt, so daß die Kugel 22 oder dergleichen die Stenose rekanalisieren kann und sich daher derart selbst einen Durchgang durch diese schaffen kann. Vorzugsweise wird die ganze Anordnung einschließlich der sich frei erstreckenden Drahtbereiche 2 bis 4 durch die Stenose hindurchgeschoben, anschließend die Aufweitung vorgenommen und durch Rückwärtsziehen der Drähte 2 bis 4 die oben erwähnte Erweiterung geschaffen.

Das abgeschälte Plaque-Material wird vorzugsweise durch den Katheter 6 abgesaugt, wobei es, wie er in der Figuren 1 und 2 dargestellt ist, neben dem Hohldraht 1 abgesaugt werden kann. Grundsätzlich kann auch ein zweilumiger Katheter mit einem Nebenlumen verwendet werden, durch welches Streptokinase oder dergleichen zum Anlösen von Ablagerungen eingespritzt werden kann.

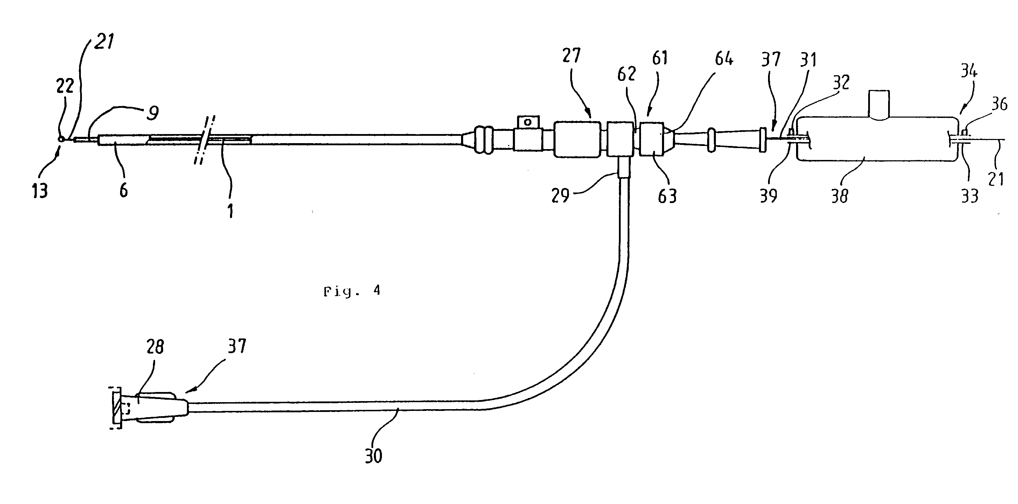

Eine Katheteranordnung 6, wie sie im Rahmen der Erfindung eingesetzt werden kann, ist in der Figur 4 dargestellt. Der Katheter 6 weist in seinem distalen Bereich 27 einen Abzweig 29 auf. An dem Abzweig 29 ist ein Schlauchteil 30 befestigt, das mit seinem dem Abzweig 29 abgewandten Ende 37 über einen Adapter 28 mit einer Absaugeinrichtung verbindbar ist, um die abgeschnittenen Ablagerungen durch den Katheter 6 abzusaugen. In bevorzugter Ausgestaltung ist die Absaugeinrichtung eine motorisch, insbesondere elektrisch, vorzugsweise batteriebetriebene Pumpe, deren Absaugrate veränderbar, wie einstellbar, steuerbar oder regelbar ist.

Proximal des Abzweigs 29 ist ein dichter Durchlaß für die Drähte 1, 21 vorgesehen. Die Dichtung ist dabei vorzugsweise derart ausgebildet, daß in einem Ventilteil 61, wie eines hämostatischen Ventils, ein kurzes, elastisches und flexibles Schlauchteil angeordnet ist, dessen Durchmesser im unbelasteten Zustand derart ist, daß der Hohldraht 1 und auch der Zugdraht 21 mit seiner Kugel 22 im unbelasteten Zustand des Schlauchteils durch dieses frei hindurchgesteckt werden kann. Ein Hülsenansatz 62 ist mit einem Gewinde versehen. Auf diesem sitzt eine mit einem Innengewinde versehene Kappe 63 auf. Die Kappe 63 drückt mit ihrer Stirnseite 64, wenn sie zum Abzweig 29 hingeschraubt wird, das Schlauchstück axial zusammen, wodurch dieses sich am Hohldraht 1 anlegt und eine sichere, zuverlässige Abdichtung bildet. Der Hohldraht 1 kann dann weiterhin unter Reibung axial vor- und zurückgeschoben werden.

Ein distales Ende 31 des Hohldrahtes 1 ist in einer angetriebene Hohlwelle 39 eines Antriebsmotors 38 axial verschiebbar, kann aber mit der Welle axial- und drehfest verspannt werden, beispielsweise über eine Verspanneinrichtung 32. Die Hohlwelle 39 ist im Motorteil 38 drehantreibbar gelagert. Entweder sie oder eine weitere Hohlwelle 33 ragt am rückwärtigen Ende 34 des Motors 38 aus diesem heraus. Durch die Hohlwellen 39, 33 erstreckt sich der Zugdraht 21, der über eine Klemmeinrichtung 36 drehfest mit der Welle 33 (die wie gesagt gegebenenfalls einstückig mit der Welle 39 ausgebildet sein kann) verbunden wird. Nach Einbringen der erfindungsgemäßen Anordnung in das freizulegende Gefäß und mit der Kugel 22 oder dergleichen bis vor den Verschluß wird der Motor 38 eingeschaltet, so daß der Zugdraht 21 (gegebenenfalls auch der Hohldraht 1) und insbesondere die mit dem Zugdraht 21 verbundene gegebenenfalls aufgerauhte Kugel 22 in Drehungen versetzt wird. Die Kugel 22 wird dann durch Vorbewegen des Motors 38 in proximaler Richtung unter ihrer durch die Drehung bewirkten Schneidwirkung gegen den Verschluß und durch diesen hindurchgedrückt, bis eine Rekanalisation erreicht ist. Anschließend wird der Hohldraht 1 mit seinem vorderen Hohldrahtansatz 9 sowie den Einzeldrahtbereichen 2 bis 4 durch den Kanal des zur proximalen Seite der Stenose hindurchgeschoben. Daraufhin kann die Klemmeinrichtung 38 gelöst werden. Der Zugdraht 21 wird zurückgezogen, so daß eine Relativverschiebung des Zugdrahtes 21 zum Hohldraht 1 bewirkt wird. Der Zugdraht 21 nimmt über die Kugel 21 den Hohldrahtansatz 9 mit, wodurch die mit den Schneiden 16 versehenen Einzeldrähte 2, 3 und 4 radial nach außen gedrückt werden. Nach Erreichen eines gewünschten Radius wird die Klemmvorrichtung 36 wieder festgelegt und anschließend wird der Motor 38 in Betrieb gesetzt, so daß sich das durch die Einzeldrähte 2 bis 4 gebildete Körbchen dreht und unter Zurückziehen des Motors 38 und damit der beiden Drähte 1 und 21 einen gewünschten Hohlzylinder aus der Stenose herausschneidet.

Dieser Vorgang kann gegebenenfalls mehrmals wiederholt werden, bis der gewünschte Durchmesser im Gefäß freigelegt ist.

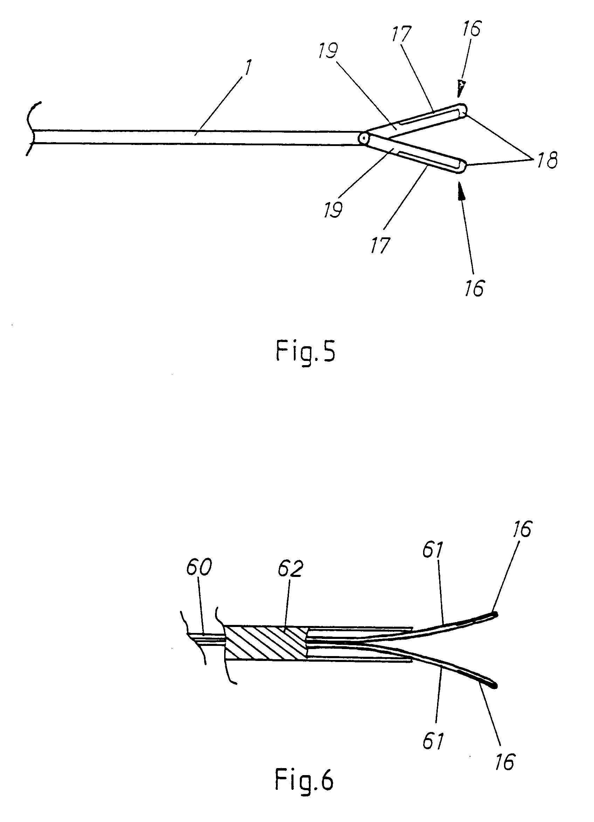

In Figur 5 ist eine zweite Ausführungsform dargestellt. Am proximalen Ende des Hohldrahts 1 ist eine zangenartige Vorrichtung angeordnet, die über einen im Inneren des Hohldrahtes 1 liegenden Draht oder dergleichen in bekannter Weise zum Öffnen und Schließen der Zange steuerbar ist. In der vereinfachten Darstellung (Figur 5) weist die Zange zwei Zangenbacken 19 mit Schneiden 16 auf. In dieser Ausführung sind die Außenkanten der Backen 19 angeschliffen und sie bilden die Außenschneiden 17, außerdem sind die Frontkanten der Backen 19 angeschliffen zur Bildung der Frontschneiden 18.

Diese Vorrichtung wird vorzugsweise so eingesetzt, daß die Zange im geschlossenen Zustand durch den Einführungskatheter 6 bis zur Stenose vorgeschoben wird. Über den Hohldraht 1 wird die Zange in Rotation versetzt und beim weiteren Vorschieben mittels des Hohldrahtes 1 können die Frontschneiden 18 die Stenose durchdringen. Die Zange wird dann in bekannter Weise, z.B. durch den Zugdraht 21 gesteuert, auf den gewünschten Durchmesser gespreizt und unter Rotation zurückgezogen. Dabei wird die Stenose durch die Außenschneiden 17 auf den eingestellten Durchmesser ausgeschält.

In Figur 6 ist eine weitere Ausführungsform dargestellt. Diese basiert auf einer Art "Silverman-Nadel" 60, die zwei Enden 61 mit Schneiden 16 aufweist. Die beiden Enden 61 haben eine innere Spannung, so daß diese sich so weit spreizen, wie es in dieser Ausführung durch die verschiebbare Hülse 62 ermöglicht wird. Die Hülse 62 kann z.B. mit dem Hohldraht 1 verbunden bzw. identisch sein, und die Nadel 60 über den sonst als Zugdraht 21 benutzten inneren Draht in Rotation versetzt werden, wenn dieser mit der Nadel 60 drehfest verbunden ist. Durch relatives, axiales Verschieben der Hülse 62 gegen die Nadel 60 spreizen sich die Enden 61 aufgrund ihrer Eigenspannung auf den gewünschten Durchmesser und entsprechend den Ausführungen zu Figur 5 kann mittels der Schneiden 16 eine Stenose durch Rotation der Nadel 60 und Vor- bzw. Zurückziehen von Nadel 60 und Hülse 62 ausgehöhlt werden.

Der erfindungsgemäße Antrieb 101 weist ein Gehäuse 102 auf, in dem sich ein Elektromotor sowie eine Energiequelle, sowie eine Batterie oder ein Akkumulator befindet, der über die Öffnung 104 in das Gehäuse einschiebbar ist. Im Gehäuse befindet sich weiterhin ein Drehzahlregler für den Motor, der über einen Einstellknopf 106 steuerbar ist, so daß die Drehzahl in gewünschten Bereichen beispielsweise zwischen 0 und 2000 Umdrehungen pro Minute oder mehr einstellbar und auch veränderbar ist. Im Gehäuse ist weiterhin eine Anzeige 107.hier eine LCD-Anzeige vorgesehen, die anzeigt, wann die Energie des Energiespeichers aufgebraucht und daher ein Wechsel notwendig ist. Weiterhin ist der Antrieb 101 mit einem Ein- und Ausschalter zum Ein- und Ausschalten des Drehantriebs versehen. An der Vorderseite des Antriebs 101 ist am Gehäuse eine Brücke 109vorgesehen, die an ihrem dem Gehäuse102abgwandten Ende (bei 110) einen von der Oberseite offenen Durchbruch mit hinterschnittenen seitlichen Nuten 130 Durchbruch 120 aufweist, in die radial Flansche an einem hohlen Führungsteil, wie einem Absaugkatheter einsetzbar sind, so daß diese relativ zum Antrieb101und insbesondere dessen Gehäuse 102 insbesondere in axialer Richtung festlegbar ist.

In den von der Haltebrücke 109 für den Katheter umgebenen Raum ragt ein Anschlußteil 110 mit einer Antriebswelle 114, die an ihrem freien Ende mit einem Verbindungsansatz 116, wie beispielsweise einem männlichen Luer-Lok versehen ist. Hier ist über das weibliche Luer-Teil eine Einrichtung zum Entfernen der Ablagerungen durch Drehen dieser Einrichtung, wie eine Rotationsspirale, drehfest anbringbar. Die genannte Einrichtung oder Rotationsspirale weist über ihre etwa der Länge des hohlen Führungsteils oder Katheters entsprechenden Länge eine gerade Welle auf und kann an ihrem proximalen Ende, das heißt dem dem Antrieb 101 abgewandten Ende mit einer Wendelausbildung sowie an dieser anschließend einer Kugel mit oder ohne Schneidkantenausbildung oder aber auch mit einem radial spreizbaren Körbchen, gegebenenfalls mit Schneidenausbildung versehen sein.

Auf der Oberseite des Gehäuses befindet sich ein Schieberteil 117, mit welchem zu diesem drehbar, aber axial fest die Welle 114 verbunden ist. Die Welle 114 steht wiederum in axialem, aber drehfesten Eingriff mit einem Abtriebsstummel des im Gehäuse 102 befindlichen Motors.

Eine konkrete Ausführungsform dieser Verbindung ist in der Figur 9 dargestellt. Die Antriebswelle 114 trägt an ihrem vorderen Ende drehfest den Luer-Lok 116. Sie weist ebenfalls drehfest mit Abstand zueinander angeordnete Radialflansche 141, 142 auf, die beispielsweise an einer aufgesetzten Hülse 143, wie einer mit der Welle 114 festgelöteten Messinghülse ausgebildet sein können. Zwischen den Flanschen 141,142 greift an der Welle114 oder der mit dieser verbundenen Hülse 143 unter Gewährleistung der Drehbeweglichkeit ein C-förmiges Teil 144 an, das über die Welle 114 beziehungsweise die Hülse 143 geschnappt wurde und fest mit dem Schieberteil verbunden, beispielsweise mit diesem einstellig ausgebildet ist.

Jenseits den Flanschen 141,142 ragt die Welle 114 in eine Führungshülse145, die über ein Lager 146 drehbar relativ zum Gehäuse gelagert ist. Am Ende der Führungshülse ragt die Abtriebswelle 147 eines Motors 148 in die Hülse 145 und ist durch eine durch Querbohrungen 149 mittels eines Stifts 150 drehfest mit dieser verbunden. Der Querschnitt des in die Führungshülse 145 ragenden Teils 114a der Welle 114 sowie der Innenquerschnitt der Führungshülse 145 sind nicht kreisförmig, sondern habe eine von der Kreisform abweichende Form, beispielsweise eine Abflachung, so daß die Welle 114 von der durch den Motor 148 angetriebenen Führungshülse 145 mitgedreht werden kann. Die Welle 114 wird weiterhin noch durch ein Lager 150a, relativ zu dem sie verschiebbar und drehbar ist, geführt.

Durch die beschriebene Ausgestaltung ist einerseits der Drehantrieb der Welle 114 durch den Motor 148 und andererseits die axiale Verschiebbarkeit der Welle 114 relativ zu und in der Hülse 145 gesichert.

In der Figur 10 ist schematisch eine Ausführungsform zur Umsetzung des vom Drehantrieb für das Anschlußteil 114 abgeleiteten linearen Hin- und Herbewegens für dieses dargestellt. Das Anschlußteil 114 ist drehbeweglich, aber axial fest mit einer Hülse 151 - ähnlich der der erwähnten Hülse 143 - verbunden, beispielsweise durch beidseitig der Hülse 151 fest auf dem Anschlußteil 114 aufsitzenden Radialflansche 152. Um die Reibung zu vermindern, kann im Inneren der Hülse 151 zwischen dieser und dem Anschlußteil 114 eine übliche Lagerung ausgebildet sein. Im Außenumfang der Hülse befindet sich eine geschlossene Nut, die mäanderförmig von der einen Stirnseite der Hülse und um diese weiter herum wieder zur ersten Stirnseite zurückgeführt ist. In die Nut greift der Nocken 156 einer weiteren Drehhülse 157 ein. Damit die Hülse 151 sich nicht bei Drehung der Hülse 157 mitdreht, ist sie über einen Nocken 161 in einer Nut 162 der Wandung 163 des Gehäuses 102 oder eines mit diesem fest verbundenen Teils geführt. Bei Drehung der Hülse 157 drängt der Nocken 156 gegen die zu seiner Drehrichtung schräg verlaufende Wandung der Nut 153 der Hülse 151. Da diese sich aufgrund ihrer Linearführung 161, 162 nicht mitdrehen kann, wird sie durch den Nocken 156 axial verschoben, bis der Nocken 156 zu einem der Umkehrpunkte der Nut 153 bei den Stirnbereichen der Hülse 151 gelangt, wo die Linearbewegung dann umgedreht wird. Über die Flansche 152 nimmt die Hülse 151 das Anschlußteil 114 bei ihrer Hin- und Herbewegung mit. Der Drehantrieb der Drehhülse 157 kann vom Drehantrieb des Motors abgeleitet werden und zwar, da die Hin- und Herbewegung der Hülse 151 und damit die Drehbewegung der Hülse 157 wesentlich geringer sein soll als die Drehbewegung des Motors über ein Untersetzungsgetriebe, das beispielsweise in Form von Sonnenrad-Planeten-Getrieben mit in der Hülse 157 ausgebildeten Innenzahnrad, in Form von Uhrwerksgetrieben oder dergleichen ausgebildet sein kann. Der Drehantrieb der Welle 114 und damit des Anschlußteils 110 erfolgt in der gleichen Weise direkt, wie dies unter Bezugnahme auf Figur 9 beschrieben wurde.

## Patentansprüche

1. Vorrichtung zur Atherektomie, mit einem Katheter und einem durch diesen hindurchgeführten länglichen Teil aus einem Hohlelement und einem sich durch dieses hindurch erstreckenden Zugdraht, wobei am proximalen Ende des Hohlelements ein drehbares Arbeitselement angeordnet ist, wobei das Hohlelement (1) an seinem distalen Ende zum Drehantrieb des mit ihm verbundenen Arbeitselements (2, 3, 4) mit einem motorischen Drehantrieb (38) versehbar ist, dadurch gekennzeichnet, daß das Arbeitselement ein aus einer achsnahen Stellung radial spreizbares Element (2, 3, 4) ist, daß der sich durch das Hohlelement (1) hindurch erstreckende Zugdraht (21) am spreizbaren Element (2, 3, 4) zum Spreizen desselben vom distalen Ende des Zugdrahtes (21) her angreift, daß das Hohlelement (1) ein schraubenförmig gewickelter Hohldraht ist und daß das drehbare Element (2, 3, 4) einstückig mit den Hohldrahtteilen (1, 9) ausgebildet ist.

2. Vorrichtung nach Anspruch 1, dadurch gekennzeichnet, daß Endbereiche des drehbaren Elements (2, 3, 4) in der radialen Spreizung axial aufeinander zu bewegbar sind.

3. Vorrichtung nach Anspruch 1 oder 2, dadurch gekennzeichnet, daß die drehbaren Elemente Drahtabschnitte (2, 3, 4) sind, die an mit Abstand voneinander angeordneten Verbindungsstellen (7, 8) zusammengehalten sind, und daß die Verbindungsstellen (7, 8) axial aufeinander zu bewegbar sind, wodurch die Drahtabschnitte (2, 3, 4) insbesondere in ihren mittleren Bereichen spreizbar sind.

4. Vorrichtung nach Anspruch 3, dadurch gekennzeichnet, daß der Zugdraht (21) durch die Verbindungsstellen (7, 8) geführt ist und durch ihn die Verbindungsstellen (7, 8) zueinander hinziehbar sind.

5. Vorrichtung nach Anspruch 3, dadurch gekennzeichnet, daß die Drahtabschnitte (2, 3, 4) im Bereich der Verbindungsstellen (7, 8) durch Hülsen (11, 12) zusammengehalten sind.

6. Vorrichtung nach einem der Ansprüche 1 bis 5, dadurch gekennzeichnet, daß sich an das drehbare Element bzw. die Drahtabschnitte (2, 3, 4) proximal ein schraubenförmig gewickelter Hohldrahtansatz (9) anschließt.

7. Vorrichtung nach einem der Ansprüche 1 bis 6, dadurch gekennzeichnet, daß die drehbaren Elemente (2, 3, 4) Schneiden (16) aufweisen.

8. Vorrichtung nach Anspruch 7, dadurch gekennzeichnet, daß die Schneiden (16) durch Anschleifen der Einzeldrähte (2, 3, 4) gebildet sind.

9. Vorrichtung nach Anspruch 8, dadurch gekennzeichnet, daß die Schneiden (16) sich nur über einen Teil (A) der Länge der Einzeldrähte (2, 3, 4) erstrecken.

10. Vorrichtung nach einem der Ansprüche 1 bis 9, dadurch gekennzeichnet, daß der Zugdraht (21) die Schneiden (16) bzw. Einzeldrähte (2, 3, 4) proximal überragt und an seinem überragenden Ende (bei 13) ein erweitertes Widerlager (Kugel 22) gegen eine distal ausgeübte Zugkraft befestigt ist.

11. Vorrichtung nach einem der Ansprüche 6 bis 10, dadurch gekennzeichnet, daß der proximale Hohldrahtansatz (9) an seinem proximalen Ende (13) mit einer Verstärkung (Hülse 14) versehen ist.

12. Vorrichtung nach Anspruch 10 oder 11, dadurch gekennzeichnet, daß das Widerlager als Kugel (22) ausgebildet ist.

13. Vorrichtung nach einem der Ansprüche 10 bis 12, dadurch gekennzeichnet, daß das Widerlager (22) glatt ist.

14. Vorrichtung nach einem der Ansprüche 10 bis 12, dadurch gekennzeichnet, daß das Widerlager (22) aufgerauht ist.

15. Vorrichtung nach einem der Ansprüche 10 bis 14, dadurch gekennzeichnet, daß das Widerlager (22) mit Schneidteilen versehen ist.

16. Vorrichtung nach einem der Ansprüche 1 bis 15, dadurch gekennzeichnet, daß Zugdraht (21) und Hohldraht (1) relativ zum motorischen Antrieb (38) axial einstellbar und festspannbar sind.

17. Vorrichtung nach einem der Ansprüche 1 bis 16, dadurch gekennzeichnet, daß der Zugdraht (21) als Hohldraht ausgebildet ist und daß sich dessen Hohlraum gegebenenfalls durch das Widerlager (22) hindurch erstreckt.

18. Vorrichtung nach Anspruch 7, dadurch gekennzeichnet, daß die Schneide(n) (16) sich in der achsnahen Stellung im wesentlichen axial erstreckt (erstrecken).

19. Vorrichtung nach Anspruch 7, dadurch gekennzeichnet, daß die Schneiden (16) zangenartig ausgebildet sind.

20. Vorrichtung nach Anspruch 19, dadurch gekennzeichnet, daß die Schneiden (16) angeschliffene Axialkanten der Zangenbacken sind.

21. Vorrichtung nach einem der vorangehenden Ansprüche, dadurch gekennzeichnet, daß der motorische Drehantrieb (38) im Bereich der distalen Enden (bei 34, 37) an dem Hohldraht (1) und/oder dem Zugdraht (21) drehfest angreift.

22. Vorrichtung nach einem der vorangehenden Ansprüche, dadurch gekennzeichnet, daß ein mit dem Drehantrieb verbundenes drehbares Anschlußteil des spreizbaren Elements in axialer Richtung relativ zur Führungsteil-Aufnahme (9) hin- und herbewegbar ist.

23. Vorrichtung nach Anspruch 22, dadurch gekennzeichnet, daß das drehbare Anschlußteil manuell axial hin- und herbewegbar ist.

24. Vorrichtung nach Anspruch 22, dadurch gekennzeichnet, daß das drehbare Anschlußteil motorisch axial hin- und herbewegbar ist.

25. Vorrichtung nach Anspruch 24, gekennzeichnet durch eine axial fest, aber drehbar mit dem Anschlußteil (10) und drehfest mit dem Gehäuse (2) des Antriebs verbundene Hülse mit einer geschlossenen, in Form eines Mäanders um den Umfang geführten Nut, in der die Nocken eines mit gegenüber der Winkelgeschwindigkeit des Anschlußteils (10) geringer Winkelgeschwindigkeit umlaufenden Übertragungsteils eingreifen.

26. Vorrichtung nach einem der Ansprüche 22 bis 25, gekennzeichnet durch eine am Anschlußteil (10) ausgebildete Aufnahme (16).

27. Vorrichtung nach einem der Ansprüche 22 bis 26, dadurch gekennzeichnet, daß im Aufnahmeteil für das Führungsteil eine einen Flansch aufnehmende, an ihren beiden Stirnseiten durch Schultern begrenzte Nut (13) ausgebildet ist.

## Claims

1. Atherectomy device having a catheter and an elongated part formed by a hollow element passed through the same and a draw wire extending through the latter and at the proximal end of the hollow element is provided a rotary working element, the hollow element (1) being provided at its distal end to the rotary drive of the working element (2, 3, 4) connected thereto with a motor rotary drive (38), characterized in that the working element is an element (2, 3, 4) radially spreadable from an axially near position, that the draw wire (21) extending through the hollow element (1) engages on the spreadable element (2, 3, 4) for spreading the latter from the distal end of the draw wire (21), that the hollow element (1) is a helically wound hollow wire and that the rotary element (2, 3, 4) is constructed in one piece with the hollow wire parts (1, 9).

2. Device according to claim 1, characterized in that end regions of the rotary element (2, 3, 4) are movable axially towards one another in the radial spreading.

3. Device according to claim 1 or 2, characterized in that the rotary elements are wire portions (2, 3, 4), which are held together at spaced connection points (7, 8) and that the connection points (7, 8) can be moved axially towards one another, so that the wire portions (2, 3, 4) are spreadable in particular in their central regions.

4. Device according to claim 3, characterized in that the draw wire (21) is guided through the connection points (7, 8) and by the same the connection points (7, 8) can be drawn towards one another.

5. Device according to claim 3, characterized in that the wire portions (2, 3, 4) are held together by sleeves (11, 12) in the vicinity of the connection points (7, 8).

6. Device according to one of the claims 1 to 5, characterized in that a helically wound hollow wire extension (9) is proximally connected to the rotary element and/or the wire portions (2, 3, 4).

7. Device according to one of the claims 1 to 6, characterized in that the rotary elements (2, 3, 4) have cutting edges (16).

8. Device according to claim 7, characterized in that the cutting edges (16) are formed by grinding the individual wires (2, 3, 4).

9. Device according to claim 8, characterized in that the cutting edges (16) extend over only part (A) of the length of the individual wires (2, 3, 4).

10. Device according to one of the claims 1 to 9, characterized in that the draw wire (21) proximally projects over the cutting edges (16) and/or individual wires (2, 3, 4) and to its projecting end (at 13) a widened abutment (ball 22) is fixed against a distally exerted tension.

11. Device according to one of the claims 6 to 10, characterized in that the proximal hollow wire extension (9) is provided at its proximal end (13) with a reinforcement (sleeve 14).

12. Device according to claim 10 or 11, characterized in that the abutment is constructed as a ball (22).

13. Device according to one of the claims 10 to 12, characterized in that the abutment (22) is smooth.

14. Device according to one of the claims 10 to 12 characterized in that the abutment (22) is roughened.

15. Device according to one of the claims 10 to 14, characterized in that the abutment (22) is provided with cutting parts.

16. Device according to one of the claims 1 to 15, characterized in that the draw wire (21) and hollow wire (1) are axially adjustable and lockable relative to the motor drive (38).

17. Device according to one of the claims 1 to 16, characterized in that the draw wire (21) is constructed as a hollow wire and its cavity optionally extends through the abutment (22).

18. Device according to claim 7, characterized in that the cutting edge or edges (16) extend substantially axially in the axially near position.

19. Device according to claim 7, characterized in that the cutting edges (16) have a tong-like construction.

20. Device according to claim 19, characterized in that the cutting edges (16) are ground axial edges of the tong jaws.

21. Device according to one of the preceding claims, characterized in that the motor rotary drive (38) in the vicinity of the distal ends (at 34, 37) engages in non-rotary manner on the hollow wire (1) and/or draw wire (21).

22. Device according to one of the preceding claims, characterized in that a rotary connecting part of the spreadable element connected to the rotary drive can be moved backwards and forwards axially relative to the guide part receptacle (9).

23. Device according to claim 22, characterized in that the rotary connecting part can be moved axially backwards and forwards in manual manner.

24. Device according to claim 22, characterized in that the rotary connecting part can be axially moved backwards and forwards in motor manner.

25. Device according to claim 24, characterized by a sleeve connected in axially fixed, but rotary manner to the connecting part (10) and in non-rotary manner to the drive casing (2) and having a closed groove circumferentially guided in the form of a meander and in which engage the cams of a transfer part rotating at a lower angular speed than the angular speed of the connecting part (10).

26. Device according to one of the claims 22 to 25, characterized by a receptacle (16) formed on the connecting part (10).

27. Device according to one of the claims 22 to 26, characterized in that in the reception part for the guide part is formed a groove (13) bounded on its two sides by shoulders and receiving a flange.

## Revendications

1. Dispositif d'athérectomie comprenant un cathéter et une partie oblongue engagée à guidage à travers celui-ci, consistant en un corps creux et un fil métallique de traction traversant ce dernier, un organe rotatif de travail étant disposé à l'extrémité proximale du corps creux, ce corps creux (1) pouvant présenter à son extrémité distale un entraînement de rotation à moteur (38) pour la rotation de l'organe de travail (2,3,4) qui lui est relié, caractérisé en ce que l'organe de travail est un organe (2,3,4) pouvant subir une extension radiale à partir d'une position proche de l'axe, en ce que le fil métallique de traction (21) traversant le corps creux (1) agit à partir de l'extrémité distale du fil métallique (21) sur l'organe (2,3,4) extensible en vue de l'extension de celui-ci, en ce que le corps creux (1) consiste en un fil métallique creux à enroulement hélicoïdal, et en ce que l'organe (2,3,4) rotatif est conformé d'une pièce avec les segments de fil métallique creux (1,9).

2. Dispositif selon la revendication 1, caractérisé en ce qu'en extension radiale, des zones d'extrémité de l'organe rotatif (2,3,4) peuvent être dirigées axialement l'une vers l'autre.

3. Dispositif selon l'une quelconque des revendications 1 ou 2, caractérisé en ce que les organes rotatifs sont des segments de fil métallique (2,3,4) reliés entre eux en des points de liaison (7,8) disposés à intervalle les uns des autres, et en ce que les points de liaison (7,8) peuvent être déplacés axialement les uns vers les autres, les segments de fil métallique (2,3,4) étant extensibles surtout dans leurs zones médianes.

4. Dispositif selon la revendication 3, caractérisé en ce que le fil métallique de traction (21) est conduit à travers les points de liaison (7,8) et que par son intermédiaire, les points de liaison (7,8) peuvent être rapprochés les uns des autres par traction.

5. Dispositif selon la revendication 3, caractérisé en ce que les segments de fil métallique (2,3,4) sont reliés entre eux dans la zone des points de liaison (7,8) par des gaines (11,12).

6. Dispositif selon l'une quelconque des revendications 1 à 5, caractérisé en ce que l'organe rotatif ou les segments de fil métallique (2,3,4) se poursuivent du côté proximal par une prolongation de fil métallique creux (9) à enroulement hélicoïdal.

7. Dispositif selon l'une quelconque des revendications 1 à 6, caractérisé en ce que les organes rotatifs (2,3,4) présentent des arêtes vives (16).

8. Dispositif selon la revendication 7, caractérisé en ce que les arêtes vives (16) sont formées par l'affûtage de chaque fil métallique (2,3,4).

9. Dispositif selon la revendication 8, caractérisé en ce que les arêtes vives (16) ne s'étendent que sur une partie (A) de la longueur de chaque fil métallique (2,3,4).

10. Dispositif selon l'une quelconque des revendications 1 à 9, caractérisé en ce que le fil métallique de traction (21) dépasse du côté proximal les arêtes vives (16) ou chaque fil métallique (2,3,4) et qu'à son extrémité en saillie (vers 13) est fixée une butée (sphère 22) à l'encontre d'une force de traction exercée du côté distal.

11. Dispositif selon l'une quelconque des revendications 6 à 10, caractérisé en ce que la prolongation de fil métallique creux (9) proximale est munie à son extrémité proximale (13) d'un renforcement (douille 14).

12. Dispositif selon l'une quelconque des revendications 10 ou 11, caractérisé en ce que la butée est conformée en sphère (22).

13. Dispositif selon l'une quelconque des revendications 10 à 12, caractérisé en ce que la butée (22) est lisse.

14. Dispositif selon l'une quelconque des revendications 10 à 12, caractérisé en ce que la butée est rugueuse.

15. Dispositif selon l'une quelconque des revendications 10 à 14, caractérisé en ce que la butée (22) est pourvue de parties coupantes.

16. Dispositif selon l'une quelconque des revendications 1 à 15, caractérisé en ce que le fil métallique de traction (21) et le fil métallique creux (1) sont réglables et rigides axialement par rapport à l'entraînement motorisé (38).

17. Dispositif selon l'une quelconque des revendications 1 à 16, caractérisé en ce que le fil métallique de traction (21) est conformé en fil métallique creux et en ce que son espace creux traverse, le cas échéant, la butée (22).

18. Dispositif selon la revendication 7, caractérisé en ce que, dans la position proche de l'axe, (les) arête(s) vive(s) (16) s'étend(ent) sensiblement axialement.

19. Dispositif selon la revendication 7, caractérisé en ce que les arêtes vives (16) sont conformées en pinces.

20. Dispositif selon la revendication 19, caractérisé en ce que les arêtes vives (16) sont des bords axiaux affûtés des becs de pince.

21. Dispositif selon l'une quelconque des revendications précédentes, caractérisé en ce que l'entraînement (38) de rotation motorisé vient en prise de rotation avec le fil métallique creux (1) et/ou le fil de traction (21) dans la zone des extrémités distales (vers 34,37).

22. Dispositif selon l'une quelconque des revendications précédentes, caractérisé en ce qu'une pièce de prolongation rotative de l'organe extensible, reliée à l'entraînement de rotation, peut être déplacée dans les deux sens dans une direction axiale par rapport au logement (9) de la pièce de guidage.

23. Dispositif selon la revendication 22, caractérisé en ce que la pièce de prolongation rotative est déplaçable axialement, manuellement, dans les deux sens.

24. Dispositif selon la revendication 22, caractérisé en ce que la pièce de prolongation rotative est déplaçable axialement, par entraînement motorisé, dans les deux sens.

25. Dispositif selon la revendication 24, caractérisé par une douille rigide axialement mais entraînée en rotation avec la pièce de prolongation et fixe en rotation par rapport au carter (2) du moteur, douille comportant une rainure périphérique fermée en forme de méandre, dans laquelle viennent en prise les ergots d'une pièce de transmission rotative dont la vitesse angulaire est faible par rapport à celle de la pièce de prolongation (10).

26. Dispositif selon l'une quelconque des revendications 22 à 25, caractérisé par un évidement (16) ménagé sur la pièce de prolongation (10).

27. Dispositif selon l'une quelconque des revendications 22 à 26, caractérisé en ce qu'une rainure (13) recevant une collerette et délimitée sur ses deux faces frontales par des épaulements est conformée dans l'évidement de l'organe de guidage.
